# EUROPEAN PATENT APPLICATION

(11) **EP 4 844 071 A1**
(43) Date of publication of application: **25.11.2026**
(21) Application number: 26753737.1
(22) Date of filing: 10.03.2026
(51) Int. Cl.: C12P 33/20, C07J 17/00

(54) **PREPARATION METHOD FOR HIGH-PURITY RARE GINSENOSIDE CK AND USE THEREOF**

(30) Priority: 10.03.2025 CN 202510276708
(71) Applicant: Guangzhou Qingnang Biotechnology Co., Ltd, Guangzhou, Guangdong 510620 (CN); Guyu Biotechnology Group Co., Ltd., Guangzhou, Guangdong 510620 (CN)
(72) Inventor: WU, Xueming, Guangzhou, Guangdong 510620 (CN); HOU, Sen, Guangzhou, Guangdong 510620 (CN); LI, Anzhang, Guangzhou, Guangdong 510620 (CN); HUANG, Zhiming, Guangzhou, Guangdong 510620 (CN); SONG, Hanliang, Guangzhou, Guangdong 510620 (CN)
(74) Representative: M. Zardi & Co S.A.
(86) International application number: PCT/CN2026/082635
(87) International publication number: WO 2026/189373

(57) **Abstract**

The present disclosure belongs to the technical field of active substance preparation, and specifically relates to a method for preparing high-purity rare ginsenoside CK and a use thereof. The present disclosure finally produces high-purity rare ginsenoside CK with a content of more than 95% through combined steps of enzymatic hydrolysis, ultrafiltration, nanofiltration, and multiple crystallization. The preparation method not only improves the production efficiency and purity of rare ginsenoside CK, but also uses only ethanol and water as solvents throughout the entire process, thereby ensuring the environmental friendliness of the process, avoiding the use of harmful chemical solvents, and greatly reducing environmental pollution. The reusability of equipment and solvents confers significant cost advantages on the entire production process, rendering it suitable for large-scale industrial application and exhibiting broad market prospects and economic benefits.

## Description

### CROSS-REFERENCE TO THE RELATED APPLICATIONS

The present disclosure claims priority to Chinese Patent Application No. 202510276708.8, filed with the China National Intellectual Property Administration on March 10, 2025, and entitled "METHOD FOR PREPARING HIGH-PURITY RARE GINSENOSIDE CK AND USE THEREOF".

### TECHNICAL FIELD

The present disclosure belongs to the technical field of active substance preparation, and specifically relates to a method for preparing high-purity rare ginsenoside (*Panax ginseng*) CK (Compound K, CAS No. 39262-14-1) and a use thereof.

### BACKGROUND

An enzymatic conversion process is currently the main production process for rare ginsenoside CK. The crude rare ginsenoside CK product obtained by enzymatic conversion has complex components, including various other saponins and unknown components. These other saponins have similar physical properties to CK, making it impossible to purify CK crude product with a content of less than 70% by relatively simple methods such as extraction, crystallization, ordinary filtration, or centrifugation. Chromatography is therefore the only option for separation and purification to obtain CK with a content of more than 80%. However, ordinary chromatography methods, such as silica gel column separation, require the use of organic solvents with higher toxicity, such as chloroform and dichloromethane. In addition, silica gel column separation has a small sample loading volume and a large elution volume. Obtaining a small amount of the target product requires using a large amount of organic solvents, resulting in high production costs and significant environmental pollution.

Patent No. CN 108138212 A discloses a process in which 2 kg of ginseng is extracted with 20 L of ethanol, followed by centrifugation, concentration under reduced pressure, decolorization with 1 L of diethyl ether, addition of 1 L of butanol, concentration under reduced pressure, addition of a small amount of ethanol, addition of a large amount of ethyl acetate, and drying of the precipitate to obtain 40-80 g of ginsenoside powder. The ginsenoside powder is subjected to enzymatic hydrolysis with pectinase at 30 °C for 72 h, concentrated with ethyl acetate, and then isolated by silica gel column chromatography to obtain CK. Disadvantages: the purification steps of this patent are cumbersome, and a variety of toxic and hazardous organic substances are used as extraction media during the preparation process, causing significant harm to the health of production personnel and the environment.

Patent No. CN105838770 A discloses a process in which 200 L of *Microbacterium oxydans* is first subjected to liquid fermentation, then RB1 with a content of 90% is dissolved in 50% ethanol to prepare 6 L of a saponin solution at 100 g/L, 10 g of heteropolyacid is added, and enzymatic hydrolysis is carried out at 30 °C for 96 h. The enzymatic hydrolysate is centrifuged to discard the supernatant, and the precipitate is rinsed. The precipitate is then extracted with water and ethyl acetate at a ratio of 1:1, the mixture is concentrated under reduced pressure, and purified and separated by preparative chromatography, with a yield of 90% and a purity of 96%. Disadvantages: this patent does not provide detailed descriptions of the purification steps. Using CK with relatively low purity for preparative chromatography separation and purification would generate a large volume of organic solvent eluent, making it difficult to obtain a substantial amount of the target product. Moreover, the application of preparative chromatography for producing materials is costly.

### SUMMARY

The present disclosure aims to overcome the shortcomings and deficiencies of the prior art by providing a method for preparing high-purity rare ginsenoside CK, which enables the production of high-purity rare ginsenoside CK having a content of more than 95%, with offering high production efficiency, reduced environmental pollution, and reusability of equipment and solvents, resulting in significant cost advantages.

To achieve the above objectives, the technical solutions adopted by the present disclosure include:
in a first aspect, the present disclosure provides a method for preparing a rare ginsenoside CK, including the following steps:
S1. mixing a ginsenoside Rb1 with a phosphate buffer solution under stirring until dissolved to obtain a ginsenoside Rb1 solution; then mixing a complex enzyme with the phosphate buffer solution to prepare a complex enzyme solution; adding the complex enzyme solution into the ginsenoside Rb1 solution for enzymatic hydrolysis, so as to obtain an enzymatic hydrolysate;
S2. centrifuging the enzymatic hydrolysate, collecting a precipitate, mixing the obtained precipitate with an ethanol solution under stirring to obtain a dissolved solution, and subjecting the dissolved solution to a pressure filtration and a microfiltration, so as to obtain a filtrate A;
S3. subjecting the filtrate A from step S2 to an ultrafiltration and a nanofiltration to obtain a concentrated filtrate B, and then drying the concentrated filtrate B, so as to obtain a dried product;
S4. mixing the dried product with anhydrous ethanol until dissolution, then adding purified water with stirring, conducting a primary recrystallization treatment, filtering to obtain crystals, and washing and drying the crystals, so as to obtain a crystalline powder; and
S5. repeating the process of step S4 with the crystalline powder for a secondary recrystallization, and drying the resulting product, so as to obtain the rare ginsenoside CK.

The present disclosure effectively improves the purification efficiency of rare ginsenoside CK through a series of process steps, including enzymatic hydrolysis, filtration, redissolution, centrifugation, microfiltration, ultrafiltration, nanofiltration, drying, and recrystallization. The entire process uses only ethanol and water as solvents, which not only ensures the environmental friendliness of the process but also avoids the use of harmful chemical solvents. In the process flow, efficient membrane filtration technology is applied, and the macromolecular and micromolecular impurities are removed from the CK crude product through ultrafiltration and nanofiltration, thus significantly improving the product purity. At the same time, by controlling the recrystallization process parameters, the purification of CK crude product, which was originally unsuitable for recrystallization, is successfully achieved. The present disclosure finally produces high-purity rare ginsenoside CK with a content of more than 95% through the combined steps of ultrafiltration, nanofiltration, and multiple crystallization. The method not only improves the production efficiency of rare ginsenoside CK but also greatly reduces environmental pollution. The reusability of equipment and solvents confers significant cost advantages on the entire production process, making it suitable for large-scale industrial application, and exhibiting broad market prospects and economic benefits.

Preferably, in the step S3, the molecular weight cut-off for the ultrafiltration is 800 to 3000 Da (Dalton).

Preferably, in the step S3, the molecular weight cut-off for the ultrafiltration is 1000 Da.

Preferably, in the step S3, the molecular weight cut-off for the nanofiltration is 100 to 300 Da.

Preferably, in the step S3, the molecular weight cut-off for the nanofiltration is 300 Da.

In the preparation method of the present disclosure, the precise regulation of the molecular weight cut-off membranes for ultrafiltration and nanofiltration is the key to improving the production efficiency and purity of rare ginsenoside CK. When the molecular weight cut-off for ultrafiltration is too low, the CK content in the ultrafiltration concentrate will be high, resulting in a low weight of the dried product after the subsequent nanofiltration, and ultimately a low overall yield. When ultrafiltration is performed at an excessively high molecular weight cut-off followed by nanofiltration, the dried powder cannot be effectively recrystallized, and a paste-like substance will be formed during the recrystallization process, which prevents effective improvement of product purity. When the molecular weight cut-off for nanofiltration is too low, the filtration speed will be slowed down, thereby significantly affecting the production efficiency of rare ginsenoside CK. If the molecular weight cut-off for nanofiltration is too high, impurities cannot be effectively removed, resulting in poor purification effects. Therefore, the present disclosure significantly improves the production efficiency and product purity by the combined ultrafiltration and nanofiltration process using the above preferred molecular weight cut-off parameter ranges for the purification of rare ginsenoside CK.

Preferably, the complex enzyme includes β-glucosidase, β-galactosidase, glucosyltransferase, and xylosidase.

Preferably, the enzyme activity of the β-glucosidase is 11000-13000 U/g, the enzyme activity of the β-galactosidase is 8000-10000 U/g, the enzyme activity of the glucosyltransferase is 3000-5000 U/g, and the enzyme activity of the xylosidase is 7000-9000 U/g. The mass ratio of the β-glucosidase, β-galactosidase, glucosyltransferase, and xylosidase is (0.05-0.3):(0.02-0.2):(0.01-0.1):(0.01-0.1).

In the process of enzymatically converting ginsenoside Rb1 into rare ginsenoside CK, β-glucosidase and β-galactosidase can effectively remove glycosyl groups, thereby simplifying the original complex sugar moieties and generating intermediate products that are more readily converted. Glucosyltransferase can adjust the affinity and reactivity of molecules by adding glycosyl groups at appropriate positions, and xylosidase can help remove impurities, thereby maintaining the purity of the reaction system.

Through experimental investigation, it has been found that when the four enzymes used in the present disclosure are combined within specific quantity ranges, the different enzymes cooperate, through different hydrolysis and transfer reactions, to precisely regulate the glycosyl structure of ginsenoside Rb1. Through synergistic effects, unnecessary glycosyl groups can be effectively removed, or glycosyl groups can be transferred to specific positions, thereby effectively improving the conversion rate of rare ginsenoside CK.

Preferably, the mass ratio of the β-glucosidase, β-galactosidase, glucosyltransferase, and xylosidase is 0.2:0.1:0.05:0.05.

Preferably, the mass ratio of the ginsenoside Rb1 to the complex enzyme is 1:(0.09-0.7).

Preferably, the mass ratio of the ginsenoside Rb1 to the complex enzyme is 1:0.4.

Preferably, in the step S4, the mass ratio of the dried product to anhydrous ethanol is 1:(15-25), and the volume ratio of the anhydrous ethanol to purified water is 1:(1.5-2.5).

Through experimental investigation, it has been found that the addition ratio of the dried product to anhydrous ethanol and water in the recrystallization process has an important influence on the final purification effect of rare ginsenoside CK. When the recrystallization process is performed using the above preferred ratio ranges, it is more conducive to improving the crystallization effect of rare ginsenoside CK, thereby effectively improving its purity. When the addition amount of water is below the above preferred range, crystals cannot be effectively formed, and crystallization fails to proceed. When the addition amount of water is above the preferred range, a paste-like precipitate is formed, resulting in a significant decrease in the purity of the target product.

Preferably, in the step S4, the mass ratio of the dried product to anhydrous ethanol is 1:20, and the volume ratio of the anhydrous ethanol to water is 1:2.

Preferably, in the step S4, the temperature for performing the primary recrystallization is -20 °C to 37 °C; in the step S5, the temperature for performing the secondary recrystallization is -20 °C to 25 °C.

Preferably, in the step S4, the temperature for performing the primary recrystallization is 4 °C; in the step S5, the temperature for performing the secondary recrystallization is 4 °C.

In the recrystallization process, temperature control also has a significant influence on the purification effect of the final target product. If the temperature is too low, although crystals form quickly, impurities in the solution are readily trapped in the crystals, leading to low product purity. If the temperature is too high, crystal formation will be slow and the crystallization amount will be small, resulting in a low yield of the final target product. Therefore, precise control of the parameter conditions in the recrystallization process is required to achieve the optimal purification effect for rare ginsenoside CK.

Preferably, in the step S4, stirring treatment is performed during the primary recrystallization, and the stirring rate is 50-150 rpm.

Preferably, in the step S4, stirring treatment is performed during the primary recrystallization, and the stirring rate is 100 rpm.

Preferably, in the step S1, the specific conditions for the enzymatic hydrolysis are: enzymatic hydrolysis at 45 °C to 55 °C for 70-72 h, at a rotation speed of 20-800 rpm, and at a pH of 6.4-6.6.

Preferably, at least one of the following (1) to (5) is included:
(1) in the step S1, the mass ratio of ginsenoside Rb1 to the phosphate buffer solution is 1:(2-20), and the concentration of the phosphate buffer solution is 0.5-1.5 mM;
(2) in the step S1, the mass ratio of the complex enzyme preparation to the phosphate buffer solution is 1:(10-20), and the concentration of the phosphate buffer solution is 0.5-1.5 mM;
(3) in the step S2, the mass concentration of the ethanol solution is 40%-95%, and the mass ratio of the precipitate to the ethanol solution is 1:(8-12);
(4) in the step S2, prior to plate-and-frame pressure filtration, the dissolved solution is mixed with diatomaceous earth under stirring, followed by the plate-and-frame pressure filtration, wherein the mass ratio of the dissolved solution to the diatomaceous earth is 1:(0.01-1); and
(5) in the step S2, the microfiltration is performed with a pore size of 0.22 µm.

Compared with the prior art, the beneficial effects of the present disclosure are as follows.
(1) In terms of purification effect, traditional purification methods often fail to effectively remove impurities, resulting in low purity. In contrast, the present disclosure effectively purifies CK crude product with a content of only 20%-70% to more than 75% by jointly employing the two-step process of ultrafiltration and nanofiltration and precisely controlling the parameter conditions in the process. Moreover, the CK crude product after ultrafiltration and nanofiltration has had impurities of both larger and smaller molecular weights removed. Through the recrystallization process, the purity can be further improved. However, CK crude product that has not been subjected to ultrafiltration and nanofiltration treatment cannot be effectively recrystallized and readily forms a paste-like precipitate during the recrystallization process. Therefore, the process of the present disclosure can effectively improve the purity and quality of the final product and is capable of achieving kilogram-scale mass production.
(2) In terms of production cost, the price of rare ginsenoside CK with a content of 90% on the current market is generally in the range of 130,000 to 400,000 RMB/kg. The preparation process described in the present disclosure can increase the content of rare ginsenoside CK to more than 95%, with the production cost per kilogram is controlled within 50,000 RMB. Thus, the production cost of high-purity rare ginsenoside CK can be significantly reduced, significantly improving the market competitiveness of the product.
(3) In terms of environmental protection, the solvents used in the preparation process described in the present disclosure include only water and ethanol, which can be recovered and reused, greatly reducing environmental pollution in the production process. Compared with conventional purification methods, the present disclosure greatly reduces the emission of harmful substances during the production process, thereby providing a feasible solution for green production.

### BRIEF DESCRIPTION OF THE DRAWINGS

FIG. 1 is an HPLC chromatogram of a ginsenoside CK reference standard;
FIG. 2 is an HPLC chromatogram of a ginsenoside CK sample prepared in Example 1;
FIG. 3 is an HPLC chromatogram of a sample before crystallization in the preparation process of Example 1; and

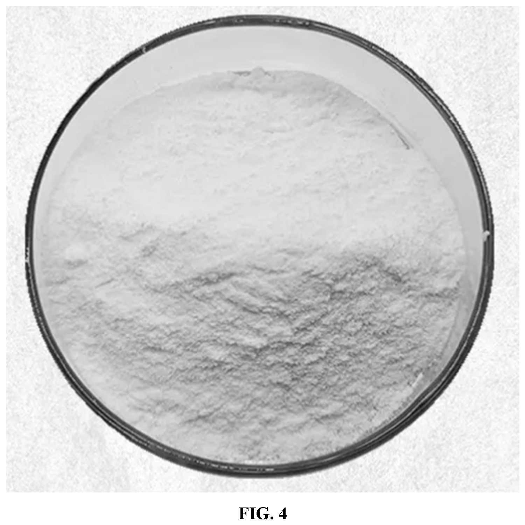

FIG. 4 is an appearance photograph of ginsenoside CK prepared in Example 1.

### DETAILED DESCRIPTION OF THE EMBODIMENTS

To better illustrate the objectives, technical solutions, and advantages of the present disclosure, the present disclosure will be further described below in conjunction with specific embodiments. Those skilled in the art should understand that the specific embodiments described herein are only used to explain the present disclosure and are not intended to limit the present disclosure.

The raw materials used in the examples and comparative examples are not specifically described and are all existing conventional raw materials available in the art, which can be purchased through commercial channels. The experimental operations not specifically described in the examples and comparative examples are all conventional operations in the art or can be understood or known by those skilled in the art based on the existing technology or common general knowledge.

### Example 1

The present example provides a method for preparing a high-purity rare ginsenoside CK, including the following steps:
S1. 1 kg of ginsenoside Rb1 powder was weighed and added to 1 mM sodium phosphate buffer (pH 7.0) at a mass ratio of 1:10. The mixture was stirred at room temperature until complete dissolution, with a stirring frequency of 200 rpm to obtain a ginsenoside Rb1 solution.
S2. β-glucosidase, β-galactosidase, glucosyltransferase, and xylosidase were mixed at a mass ratio of 0.2:0.1:0.05:0.05 to obtain a complex enzyme. The complex enzyme and 1 mM sodium phosphate buffer (pH 7.0) were mixed at a mass ratio of 1:15 until complete dissolution, so as to obtain a complex enzyme solution. The enzyme activities of β-glucosidase, β-galactosidase, glucosyltransferase, and xylosidase were measured by the DNS method to be 12000 U/g, 9000 U/g, 4000 U/g, and 8000 U/g, respectively. The mass ratio of the β-glucosidase, β-galactosidase, glucosyltransferase, and xylosidase was 0.2:0.1:0.05:0.05.
S3. Enzymatic hydrolysis: the complex enzyme solution obtained in step S2 was added to the ginsenoside Rb1 solution obtained in step S1 for enzymatic hydrolysis. After uniform stirring, the pH was adjusted to 6.5 ± 0.1 with a 10% sodium hydroxide solution. The enzymatic hydrolysis was performed for 72 h at an enzymatic hydrolysis temperature of 50 °C and a stirring frequency of 800 rpm. The mass ratio of the ginsenoside Rb1 to the complex enzyme was 1:0.4.
S4. The enzymatic hydrolysate was centrifuged at a centrifugation speed of 16,000 rpm. The supernatant was discarded, and the precipitate was collected. The precipitate was rinsed with a small amount of purified water, and then mixed with 50% ethanol solution in a reaction kettle at a mass ratio of 1:10. The mixture was stirred at 200 rpm with the temperature set to 50°C until the precipitate was completely dissolved, so as to obtain a dissolved solution.
S5. Pressure filtration: after cleaning and installing the plate-and-frame filter press, diatomaceous earth was weighed and added to the reaction kettle to mix uniformly with the dissolved solution. The mass ratio of the dissolved solution to the diatomaceous earth was 1:0.02. The discharge port of the reaction kettle was opened, and the peristaltic pump was activated to introduce the dissolved solution from the reaction kettle into the plate-and-frame filter press, so as to obtain a clear liquid.
S6. Microfiltration: a 0.22 µm PTFE pleated filter cartridge was rinsed with purified water until the pH of the liquid discharged from the outlet was approximately 7.0, a stainless steel filter was installed, the peristaltic pump was activated, the exhaust port was opened to release pressure, and the outlet of the stainless steel filter was opened to drain the residual liquid inside therein, the clear liquid obtained in step S5 was introduced, subjected to self-circulation for 5 min, and then discharged, so as to obtain filtrate A.
S7. Ultrafiltration: the ultrafiltration machine was rinsed with purified water. After the conductivity of the waste liquid discharged from the waste liquid outlet was below 5 us/cm, the filtrate A obtained in step S6 was introduced into the ultrafiltration machine for ultrafiltration. The ultrafiltration was carried out at a molecular weight cut-off of 1000 Da, and the ultrafiltration permeate was collected.
S8. Nanofiltration: the nanofiltration machine was rinsed with purified water. After the conductivity of the waste liquid discharged from the waste liquid outlet was below 5 us/cm, the ultrafiltration permeate was introduced into the nanofiltration machine for nanofiltration. The nanofiltration was carried out at a molecular weight cut-off of 300 Da, so as to obtain the concentrated filtrate B.
S9. Drying: the concentrated filtrate B was evaporated with a rotary evaporator until a paste-like state was achieved. The material was then transferred to a vacuum drying oven with the temperature set to 105 °C and the material thickness less than 1 cm. During the drying process, the surface of the material was repeatedly smoothed with a scraper. Finally, the paste-like substance was dried to a block-shaped dried product with a moisture content of less than 5%.
S10. Primary recrystallization: the dried product obtained in step S9 was mixed with anhydrous ethanol at a mass ratio of 1:20 and stirred until completely dissolved. Purified water was added and stirred uniformly. The volume ratio of the anhydrous ethanol to purified water was 1:2. The solution was placed in an environment at 4 °C and stirred with a stirrer for 24 h at a stirring rate of 100 rpm. The precipitated crystals were filtered with a Buchner funnel, which had been placed in a refrigerator at 4 °C overnight in advance. After filtration, the crystals were immediately washed with 1 L of 35% ice-cold ethanol, and the washing procedure was repeated twice with 1 L of 35% ice-cold ethanol each time by suction filtration. The crystals were then dried at 105 °C for 4 h, so as to obtain a crystalline powder.
S11. Secondary recrystallization: the above step S10 was repeated. The crystalline powder obtained from the primary recrystallization was mixed with anhydrous ethanol at a mass ratio of 1:20 and stirred until completely dissolved. Purified water was added and stirred uniformly. The volume ratio of the anhydrous ethanol to purified water was 1:2. The solution was placed in an environment at 4 °C and stirred with a stirrer for 24 h at a stirring rate of 100 rpm. The precipitated crystals were filtered with a Buchner funnel. After filtration, the crystals were immediately washed with 1 L of 35% ice-cold ethanol, and the washing procedure was repeated twice with 1 L of 35% ice-cold ethanol each time by suction filtration. The crystals were dried at 105 °C for 4 h, so as to obtain the rare ginsenoside CK.

### Example 2

The present example provides a method for preparing a high-purity rare ginsenoside CK, which is similar to the method described in Example 1, and the differences are only that:
in step S2, the mass ratio of the β-glucosidase, β-galactosidase, glucosyltransferase, and xylosidase was 0.05:0.02:0.01:0.01; and
in step S3, the mass ratio of the ginsenoside Rb1 to the complex enzyme was 1:0.09.

### Example 3

The present example provides a method for preparing a high-purity rare ginsenoside CK, which is similar to the method described in Example 1, and the differences are only that:
in step S2, the mass ratio of the β-glucosidase, β-galactosidase, glucosyltransferase, and xylosidase was 0.3:0.2:0.1:0.1; and
in step S3, the mass ratio of the ginsenoside Rb1 to the complex enzyme was 1:0.9.

### Example 4

The present example provides a method for preparing a high-purity rare ginsenoside CK, which is similar to the method described in Example 1, and the differences are only that:
in step S7, the molecular weight cut-off for ultrafiltration was 800 Da; and
in step S8, the molecular weight cut-off for nanofiltration was 100 Da.

### Example 5

The present example provides a method for preparing a high-purity rare ginsenoside CK, which is similar to the method described in Example 1, and the differences are only that:
in step S7, the molecular weight cut-off for ultrafiltration was 3000 Da.

### Example 6

The present example provides a method for preparing a high-purity rare ginsenoside CK, including the following steps:
S1. 1 kg of ginsenoside Rb1 powder was weighed and added to 1 mM sodium phosphate buffer (pH 7.0) at a mass ratio of 1:2. The mixture was stirred at room temperature until complete dissolution with a stirring frequency of 200 rpm to obtain a ginsenoside Rb1 solution.
S2. β-glucosidase, β-galactosidase, glucosyltransferase, and xylosidase were mixed at a mass ratio of 0.2:0.1:0.05:0.05 to obtain a complex enzyme. The complex enzyme and 1 mM sodium phosphate buffer (pH 7.0) were mixed at a mass ratio of 1:20 until completely dissolved, so as to obtain a complex enzyme solution. The enzyme activities of β-glucosidase, β-galactosidase, glucosyltransferase, and xylosidase were measured by the DNS method to be 12000 U/g, 9000 U/g, 4000 U/g, and 8000 U/g, respectively. The mass ratio of the β-glucosidase, β-galactosidase, glucosyltransferase, and xylosidase was 0.2:0.1:0.05:0.05.
S3. Enzymatic hydrolysis: the complex enzyme solution obtained in step S2 was added to the ginsenoside Rb1 solution obtained in step S1 for enzymatic hydrolysis. After uniform stirring, the pH was adjusted to 6.5 ± 0.1 with a 10% sodium hydroxide solution. The enzymatic hydrolysis was performed for 74 h at an enzymatic hydrolysis temperature of 45 °C and a stirring frequency of 20 rpm. The mass ratio of the ginsenoside Rb1 to the complex enzyme was 1:0.4.
S4. The enzymatic hydrolysate was centrifuged at a centrifugation speed of 16,000 rpm. The supernatant was discarded, and the precipitate was collected. The precipitate was rinsed with a small amount of purified water, and then mixed with 40% ethanol solution in a reaction kettle at a mass ratio of 1:8. The mixture was stirred at 200 rpm with the temperature set to 50 °C until the precipitate was completely dissolved, so as to obtain a dissolved solution.
S5. Pressure filtration: after cleaning and installing the plate-and-frame filter press, diatomaceous earth was weighed and added to the reaction kettle to mix uniformly with the dissolved solution. The mass ratio of the dissolved solution to the diatomaceous earth was 1:0.01. The discharge port of the reaction kettle was opened, and the peristaltic pump was activated to introduce the dissolved solution from the reaction kettle into the plate-and-frame filter press, so as to obtain a clear liquid.
S6. Microfiltration: a 0.22 µm PTFE pleated filter cartridge was rinsed with purified water until the pH of the liquid discharged from the outlet was approximately 7.0, a stainless steel filter was installed, the peristaltic pump was activated, the exhaust port was opened to release pressure, and the outlet of the stainless steel filter was opened to drain the residual liquid inside therein, the clear liquid obtained in step S5 was introduced, subjected to self-circulation for 5 min, and then discharged, so as to obtain filtrate A.
S7. Ultrafiltration: the ultrafiltration machine was rinsed with purified water. After the conductivity of the waste liquid discharged from the waste liquid outlet was below 5 us/cm, the filtrate A obtained in step S6 was introduced into the ultrafiltration machine for ultrafiltration. The molecular weight cut-off for ultrafiltration was 1000 Da, and the ultrafiltration permeate was collected.
S8. Nanofiltration: the nanofiltration machine was rinsed with purified water. After the conductivity of the waste liquid discharged from the waste liquid outlet was below 5 us/cm, the ultrafiltration permeate was introduced into the nanofiltration machine for nanofiltration. The molecular weight cut-off for nanofiltration was 300 Da, so as to obtain the concentrated filtrate B.
S9. Drying: the concentrated filtrate B was evaporated with a rotary evaporator until a paste-like state was achieved. The material was then transferred to a vacuum drying oven with the temperature set to 105 °C, and the material thickness was less than 1 cm. During the drying process, the surface of the material was repeatedly smoothed with a scraper. Finally, the paste-like substance was dried to a block-shaped dried product with a moisture content of less than 5%.
S10. Primary recrystallization: the dried product obtained in step S9 was mixed with anhydrous ethanol at a mass ratio of 1:15 and stirred until completely dissolved. Purified water was added and stirred uniformly. The volume ratio of the anhydrous ethanol to purified water was 1:1.5. The solution was placed in an environment at -20 °C and stirred with a stirrer for 24 h at a stirring rate of 150 rpm. The precipitated crystals were filtered with a Buchner funnel, which had been placed in a refrigerator at 4 °C overnight in advance. After filtration, the crystals were immediately washed with 1 L of 35% ice-cold ethanol, and the washing procedure was repeated twice with 1 L of 35% ice-cold ethanol each time by suction filtration. The crystals were then dried at 105 °C for 4, so as to obtain a crystalline powder.
S11. Secondary recrystallization: the above step S10 was repeated. The crystalline powder obtained from the primary recrystallization was mixed with anhydrous ethanol at a mass ratio of 1:15 and stirred until completely dissolved. Purified water was added and stirred uniformly. The volume ratio of the anhydrous ethanol to purified water was 1:1.5. The solution was placed in an environment at -20 °C and stirred with a stirrer for 24 h at a stirring rate of 150 rpm. The precipitated crystals were filtered with a Buchner funnel. After filtration, the crystals were immediately washed with 1 L of 35% ice-cold ethanol, and the washing procedure was repeated twice with 1 L of 35% ice-cold ethanol each time by suction filtration. The crystals were dried at 105 °C for 4 h, so as to obtain the rare ginsenoside CK.

### Example 7

The present example provides a method for preparing a high-purity rare ginsenoside CK, including the following steps:
S1. 1 kg of ginsenoside Rb1 powder was weighed and added to 1 mM sodium phosphate buffer (pH 7.0) at a mass ratio of 1:20. The mixture was stirred at room temperature until complete dissolution with a stirring frequency of 200 rpm to obtain a ginsenoside Rb1 solution.
S2. β-glucosidase, β-galactosidase, glucosyltransferase, and xylosidase were mixed at a mass ratio of 0.2:0.1:0.05:0.05 to obtain a complex enzyme. The complex enzyme and 1 mM sodium phosphate buffer (pH 7.0) were mixed at a mass ratio of 1:20 until completely dissolved, so as to obtain a complex enzyme solution. The enzyme activities of β-glucosidase, β-galactosidase, glucosyltransferase, and xylosidase were measured by the DNS method to be 12000 U/g, 9000 U/g, 4000 U/g, and 8000 U/g, respectively. The mass ratio of the β-glucosidase, β-galactosidase, glucosyltransferase, and xylosidase was 0.2:0.1:0.05:0.05.
S3. Enzymatic hydrolysis: the complex enzyme solution obtained in step S2 was added to the ginsenoside Rb1 solution obtained in step S1 for enzymatic hydrolysis. After uniform stirring, the pH was adjusted to 6.5 ± 0.1 with a 10% sodium hydroxide solution. The enzymatic hydrolysis was performed for 70 h at an enzymatic hydrolysis temperature of 55 °C and a stirring frequency of 800 rpm. The mass ratio of the ginsenoside Rb1 to the complex enzyme was 1:0.4.
S4. The enzymatic hydrolysate was centrifuged at a centrifugation speed of 16000 rpm. The supernatant was discarded, and the precipitate was collected. The precipitate was rinsed with a small amount of purified water, and then mixed with 95% ethanol solution in a reaction kettle at a mass ratio of 1:12. The mixture was stirred at 200 rpm with the temperature set to 50 °C until the precipitate was completely dissolved, so as to obtain a dissolved solution.
S5. Pressure filtration: after cleaning and installing the plate-and-frame filter press, diatomaceous earth was weighed and added to the reaction kettle to mix uniformly with the dissolved solution. The mass ratio of the dissolved solution to the diatomaceous earth was 1:0.1. The discharge port of the reaction kettle was opened, and the peristaltic pump was activated to introduce the dissolved solution from the reaction kettle into the plate-and-frame filter press, so as to obtain a clear liquid.
S6. Microfiltration: a 0.22 µm PTFE pleated filter cartridge was rinsed with purified water until the pH of the liquid discharged from the outlet was approximately 7.0, a stainless steel filter was installed, the peristaltic pump was activated, the exhaust port was opened to release pressure, and the outlet of the stainless steel filter was opened to drain the residual liquid inside therein, the clear liquid obtained in step S5 was introduced, subjected to self-circulation for 5 min, and then discharged, so as to obtain filtrate A.
S7. Ultrafiltration: the ultrafiltration machine was rinsed with purified water. After the conductivity of the waste liquid discharged from the waste liquid outlet was below 5 us/cm, the filtrate A obtained in step S6 was introduced into the ultrafiltration machine for ultrafiltration. The molecular weight cut-off for ultrafiltration was 1000 Da, and the ultrafiltration permeate was collected.
S8. Nanofiltration: the nanofiltration machine was rinsed with purified water. After the conductivity of the waste liquid discharged from the waste liquid outlet was below 5 us/cm, the ultrafiltration permeate was introduced into the nanofiltration machine for nanofiltration. The molecular weight cut-off for nanofiltration was 300 Da, so as to obtain the concentrated filtrate B.
S9. Drying: the concentrated filtrate B was evaporated with a rotary evaporator until a paste-like state was achieved. The material was then transferred to a vacuum drying oven with the temperature set to 105 °C and the material thickness less than 1 cm. During the drying process, the surface of the material was repeatedly smoothed with a scraper. Finally, the paste-like substance was dried to a block-shaped dried product with a moisture content of less than 5%.
S10. Primary recrystallization: the dried product obtained in step S9 was mixed with anhydrous ethanol at a mass ratio of 1:25 and stirred until completely dissolved. Purified water was added and stirred uniformly. The volume ratio of the anhydrous ethanol to purified water was 1:2.5. The solution was placed in an environment at 37 °C and stirred with a stirrer for 24 h at a stirring rate of 20 rpm. The precipitated crystals were filtered with a Buchner funnel, which had been placed in a refrigerator at 4 °C overnight in advance. After filtration, the crystals were immediately washed with 1 L of 35% ice-cold ethanol, and the washing procedure was repeated twice with 1 L of 35% ice-cold ethanol each time by suction filtration. The crystals were then dried at 105 °C for 4 h, so as to obtain a crystalline powder.
S11. Secondary recrystallization: the above step S10 was repeated. The crystalline powder obtained from the primary recrystallization was mixed with anhydrous ethanol at a mass ratio of 1:25 and stirred until completely dissolved. Purified water was added and stirred uniformly. The volume ratio of the anhydrous ethanol to purified water was 1:2.5. The solution was placed in an environment at 25 °C and stirred with a stirrer for 24 h at a stirring rate of 20 rpm. The precipitated crystals were filtered with a Buchner funnel. After filtration, the crystals were immediately washed with 1 L of 35% ice-cold ethanol, and the washing procedure was repeated twice with 1 L of 35% ice-cold ethanol each time by suction filtration. The crystals were dried at 105 °C for 4 h, so as to obtain the rare ginsenoside CK.

### Comparative Example 1

The present comparative example provides a method for preparing a high-purity rare ginsenoside CK, which is similar to the method described in Example 1, and the differences are only that:
β-galactosidase was not added, and the mass ratio of the β-glucosidase, glucosyltransferase, and xylosidase was 0.2:0.1:0.1. The mass ratio of the ginsenoside Rb1 to the complex enzyme remained 1:0.4.

### Comparative Example 2

The present comparative example provides a method for preparing a high-purity rare ginsenoside CK, which is similar to the method described in Example 1, and the differences are only that:
glucosyltransferase was not added, and the mass ratio of the β-glucosidase, β-galactosidase, and xylosidase was 0.2:0.1:0.1. The mass ratio of the ginsenoside Rb1 to the complex enzyme remained 1:0.4.

### Comparative Example 3

The present comparative example provides a method for preparing a high-purity rare ginsenoside CK, which is similar to the method described in Example 1, and the differences are only that:
in step S7, the molecular weight cut-off for ultrafiltration was 500 Da; and
in step S8, the molecular weight cut-off for nanofiltration was 50 Da.

### Comparative Example 4

The present comparative example provides a method for preparing a high-purity rare ginsenoside CK, which is similar to the method described in Example 1, and the differences are only that:
in step S7, the molecular weight cut-off for ultrafiltration was 5000 Da; and
in step S8, the molecular weight cut-off for nanofiltration was 400 Da.

### Comparative Example 5

The present comparative example provides a method for preparing a high-purity rare ginsenoside CK, which is similar to the method described in Example 1, and the differences are only that: in the present comparative example, the ultrafiltration process was not performed, and the filtrate A obtained in step S6 was directly subjected to subsequent nanofiltration treatment.

### Comparative Example 6

The present comparative example provides a method for preparing a high-purity rare ginsenoside CK, which is similar to the method described in Example 1, and the differences are only that: in the present comparative example, the nanofiltration process was not performed, and the ultrafiltration permeate obtained in step S7 was directly subjected to subsequent drying treatment.

### Comparative Example 7

The present comparative example provides a method for preparing a high-purity rare ginsenoside CK, which is similar to the method described in Example 1, and the differences are only that:
in step S10, the volume ratio of the anhydrous ethanol to purified water was 1:3.5, and the solution was placed in an environment at -30 °C for recrystallization.

### Comparative Example 8

The present comparative example provides a method for preparing a high-purity rare ginsenoside CK, which is similar to the method described in Example 1, and the differences are only that:
in step S10, the volume ratio of the anhydrous ethanol to purified water was 1:0.5, and the solution was placed in an environment at 45 °C for recrystallization.

### Comparative Example 9

The present comparative example provides a method for preparing a rare ginsenoside CK, which is similar to the method described in Example 1, and the differences are only that: in the present comparative example, the secondary recrystallization process was not performed.

### Effect Example

The rare ginsenoside CK prepared by the preparation methods of Examples 1 to 7 and Comparative Examples 1 to 9 were used as samples, and the ginsenoside CK content and conversion rate were detected and calculated by liquid chromatography. The specific operation process of liquid chromatography detection is as follows:
25 mg of ginsenoside CK reference standard was weighed (accurate to 0.0001 g), placed in a 25 mL volumetric flask, and an appropriate amount of methanol was added for dissolution. Methanol was continuously added to reach the scale mark. The mixture was shaken uniformly to obtain a standard solution with a concentration of 1000 mg/L (stock solution), which was stored sealed at 4 °C and could be preserved for one month. The purity of the ginsenoside CK reference standard was not less than 98%.

Aliquots of 0.50 mL, 1.0 mL, 2.5 mL, and 5 mL of the stock solution of ginsenoside CK reference standard were transferred to 10 mL volumetric flasks and made up to the mark with methanol, so as to obtain ginsenoside CK standard series solutions having corresponding concentrations of 50 mg/L, 100 mg/L, 250 mg/L, 500 mg/L, and 1000 mg/L.

For a solid powder test sample with a ginsenoside CK content of not less than 10%, 0.2-0.5 g was weighed (accurate to 0.0001 g); for a liquid sample with a content of less than 10%, 2-10 g was weighed (accurate to 0.0001 g). For samples with lower ginsenoside CK content, the sample weight could be appropriately increased. The sample was placed in a 25 mL volumetric flask, made up to the mark with methanol, subjected to ultrasonic oscillation for 30 minutes, shaken uniformly, and then filtered through a 0.22 µm organic filter membrane, so as to form a sample to be tested. When the content in the sample fell outside the standard curve range, the sample was appropriately diluted, and a suitable standard curve range was selected for detection.

The chromatographic separation was performed on a C18 column, the detection wavelength was 311 nm, the flow rate was 1.0 mL/min, the column temperature was 35 °C, and the injection volume was 10 µL. The mobile phase A was first-grade water conforming to GB/T 6682, and the mobile phase B was chromatographic grade acetonitrile. Gradient elution was performed according to the program shown in Table 1. The standard series solutions were sequentially injected, and the chromatograms were recorded. A standard curve was plotted with the standard series solution concentration as the abscissa and the peak area as the ordinate. The samples to be tested were then sequentially injected, and the chromatograms were recorded. Qualitative analysis was performed based on retention time and UV spectra, and the peak area was measured. The mass concentration of each component in the solution to be tested was obtained according to the standard curve, and the content and conversion rate of each component in the sample were calculated according to the following formula: $ω = \frac{ρ \times V \times n \times {10}^{- 4}}{m} ,$ wherein
in the formula, ω is the mass fraction of each component in the sample, %; ρ is the mass concentration of each component to be tested obtained from the standard curve, mg/L; V is the volume to which the sample was made up, mL; m is the sample amount, g; and n is the dilution factor. $Conversion rate \left(%\right) = \frac{n \left(ginsenoside CK\right)}{n \left(ginsenoside Rb1\right)} \times 100 % ,$ wherein
in the above formula 2, n is the amount of substance of ginsenoside CK or ginsenoside Rb1, and the amount of substance is obtained by multiplying the content of ginsenoside by the weight of ginsenoside, and then dividing by the relative molecular mass of ginsenoside (the relative molecular mass of ginsenoside Rb1 is 1109.29, and the relative molecular mass of ginsenoside CK is 622.87).

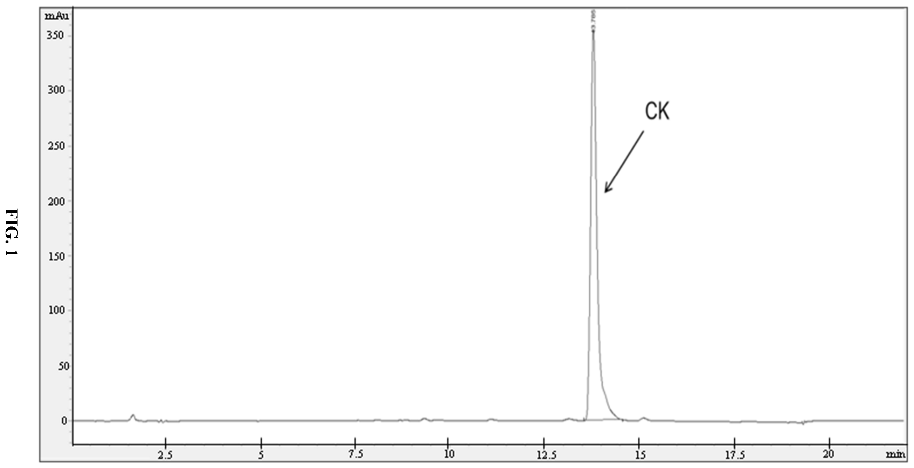

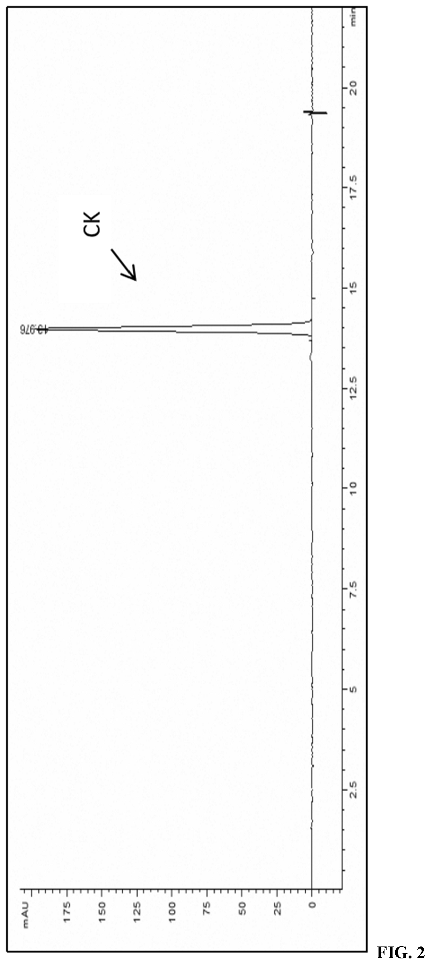

The liquid chromatogram of the ginsenoside CK reference standard is shown in FIG. 1, and the liquid chromatogram of the ginsenoside CK prepared in Example 1 is shown in FIG. 2. The specific results of the content and conversion rate of each component in the samples are shown in Table 2.

**Table 1 Elution Program for Liquid Chromatography Detection of Ginsenoside CK**

| Time/min | Volume (Mobile Phase A)/% | Volume (Mobile Phase B)/% |
|---|---|---|
| 0 | 80 | 20 |
| 4 | 50 | 50 |
| 12 | 20 | 80 |
| 16 | 0 | 100 |
| 16.5 | 60 | 40 |
| 17 | 80 | 20 |
| 20 | 80 | 20 |

**Table 2**

| Group | Weig ht of Rb1 (g) | Content of Ginsenos ide Rb1 (%) | Weight of Ginsenosi de CK after Primary Crystalliza tion (g) | Content of Ginsenosi de CK after Primary Crystalliza tion (%) | Weight of Ginsenosi de CK after Secondary Crystalliza tion (g) | Content of Ginsenosi de CK after Secondary Crystalliza tion (%) | Conversi on Rate of Ginsenos ide Rb1 to CK (%) |
|---|---|---|---|---|---|---|---|
| Example 1 | 1000 | 70.9 | 320 | 87.57 | 253 | 97.16 | 61.75 |
| Example 2 | 1000 | 70.9 | 283 | 79.22 | 236 | 84.13 | 49.87 |
| Example 3 | 1000 | 70.9 | 321 | 87.03 | 251 | 96.92 | 61.11 |
| Example 4 | 1000 | 71.78 | 308 | 81.18 | 237 | 90.1 | 53.64 |
| Example 5 | 1000 | 71.78 | 326 | 80.72 | 246 | 90.12 | 55.69 |
| Example 6 | 1000 | 71.64 | 294 | 79.58 | 224 | 88.82 | 49.98 |
| Example 7 | 1000 | 71.64 | 277 | 78.89 | 216 | 87.91 | 47.70 |
| Compara tive Example 1 | 1000 | 70.9 | 151 | 62.86 | 129 | 65.37 | 21.18 |
| Compara tive Example 2 | 1000 | 70.9 | 153 | 61.75 | 128 | 64.28 | 20.67 |
| Compara tive Example 3 | 1000 | 70.9 | 316 | 72.08 | 228 | 77.39 | 44.32 |
| Compara tive Example 4 | 1000 | 70.9 | 279 | 71.96 | 205 | 76.37 | 39.33 |
| Compara tive Example 5 | 1000 | 71.78 | 308 | 70.11 | 219 | 70.36 | 38.71 |
| Compara tive Example 6 | 1000 | 71.78 | 311 | 68.29 | 238 | 69.14 | 41.33 |
| Compara tive Example 7 | 1000 | 71.64 | 307 | 76.19 | 215 | 79.72 | 43.05 |
| Compara tive Example 8 | 1000 | 71.64 | 301 | 75.67 | 209 | 78.85 | 41.40 |
| Compara tive Example 9 | 1000 | 71.64 | 318 | 87.1 | / | / | / |

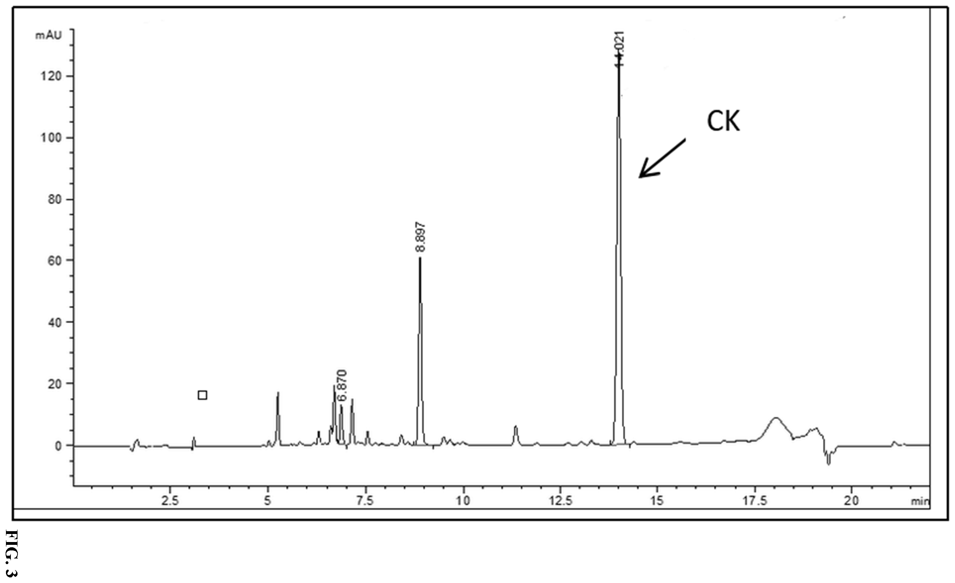

It can be seen from the results in Table 2 and FIGS. 1 to 3 that the preparation methods described in Examples 1 to 7 of the present disclosure can effectively improve the production efficiency and purity of rare ginsenoside CK. In particular, the method described in Example 1 can increase the content of ginsenoside CK in the product to 97.16%, achieving the best purification effect of ginsenoside CK. In Comparative Examples 1 to 2, when β-galactosidase and glucosyltransferase in the specific complex enzyme are each missing, the conversion rate of ginsenoside CK is significantly reduced, indicating that the four enzymes selected in the present disclosure cooperate with each other to better precisely regulate the glycosyl moieties of ginsenoside Rb1 and synergistically improve the conversion rate of ginsenoside CK. In Comparative Examples 3 to 4, when the molecular weight cut-off parameter conditions in the ultrafiltration and nanofiltration processes exceed the preferred ranges of the present disclosure, the purity of ginsenoside CK is also significantly reduced. In Comparative Examples 5 to 6, when the ultrafiltration and nanofiltration processes are each missing, the purity of the obtained ginsenoside CK decreases more significantly. The above results show that the present disclosure achieves better production efficiency and purity of ginsenoside CK through the combined ultrafiltration and nanofiltration processes for purifying ginsenoside, and by precisely regulating the key parameter of the molecular weight cut-off in the process. Once the molecular weight cut-off deviates slightly from the scope defined in the present disclosure, the purification effect of ginsenoside CK becomes significantly worse. In Comparative Examples 7 to 9, the important parameters involved in the recrystallization process of the preparation method described in the present disclosure were investigated. It was found that the recrystallization temperature and the amount of water added during crystallization both affect the final purification effect of ginsenoside CK, and that a single recrystallization process is insufficient to sufficiently improve the purity. Therefore, the present disclosure proposes a strategy combining multiple processes to improve the purity and stability of the final product.

It should be noted that the above examples are only used to illustrate the technical solutions of the present disclosure and are not intended to limit the scope of protection of the present disclosure. Although the present disclosure is described in detail with reference to the preferred examples, those of ordinary skill in the art should understand that modifications or equivalent substitutions can be made to the technical solutions of the present disclosure without departing from the essence and scope of the technical solutions of the present disclosure.

### INDUSTRIAL APPLICABILITY

The preparation method provided by the present disclosure not only improves the production efficiency and purity of rare ginsenoside CK, but also features an environmentally friendly preparation process, avoids the use of harmful chemical solvents, and greatly reduces environmental pollution, In addition, the reusability of equipment and solvents confers significant cost advantages on the entire production process, rendering it suitable for large-scale industrial application and endowing it with prospects for industrial application.

## Claims

1. A method for preparing a rare ginsenoside CK, comprising the following steps:
S1. mixing a ginsenoside Rb1 with a phosphate buffer solution and stirring until dissolved to obtain a ginsenoside Rb1 solution; then mixing a complex enzyme with the phosphate buffer solution to prepare a complex enzyme solution; adding the complex enzyme solution to the ginsenoside Rb1 solution for enzymatic hydrolysis to obtain an enzymatic hydrolysate;
S2. centrifuging the enzymatic hydrolysate, collecting a precipitate, and mixing the obtained precipitate with an ethanol solution and stirring to obtain a dissolved solution, and subjecting the dissolved solution to a pressure filtration and a microfiltration to obtain a filtrate A;
S3. Subjecting the filtrate A described in step S2 to an ultrafiltration and a nanofiltration to obtain a concentrated filtrate B, and subjecting the concentrated filtrate B to a drying treatment to obtain a dried product;
S4. mixing the dried product with anhydrous ethanol until dissolved, then adding purified water and stirring uniformly, performing a primary recrystallization treatment, filtering to obtain crystals, and washing and drying the crystals to obtain a crystalline powder; and
S5. repeating the process of step S4 with the crystalline powder to perform a secondary recrystallization, and drying to obtain the rare ginsenoside CK;
wherein the complex enzyme comprises β-glucosidase, β-galactosidase, glucosyltransferase, and xylosidase.

2. The method according to claim 1, wherein an enzyme activity of the β-glucosidase is 11000 to 13000 U/g, an enzyme activity of the β-galactosidase is 8000 to 10000 U/g, an enzyme activity unit of the glucosyltransferase is 3000 to 5000 U/g, and an enzyme activity of the xylosidase is 7000 to 9000 U/g; and
a mass ratio of the β-glucosidase, β-galactosidase, glucosyltransferase, and xylosidase is (0.05 to 0.3):(0.02 to 0.2):(0.01 to 0.1):(0.01 to 0.1).

3. The method according to claim 1, wherein a mass ratio of the ginsenoside Rb1 to the complex enzyme is 1:(0.09 to 0.7).

4. The method according to claim 1, wherein in the step S3, a molecular weight cut-off for the ultrafiltration is 800 Da to 3000 Da.

5. The method according to claim 1, wherein in the step S3, a molecular weight cut-off for the nanofiltration is 100 Da to 300 Da.

6. The method according to claim 1, wherein in the step S4, a mass ratio of the dried product to anhydrous ethanol is 1:(15 to 25), and a volume ratio of the anhydrous ethanol to purified water is 1:(1.5 to 2.5).

7. The method according to claim 1, wherein in the step S1, specific conditions for the enzymatic hydrolysis are: performing the enzymatic hydrolysis at 45 °C to 55 °C for 70 to 74 h, at a rotation speed of 20 to 800 rpm, and at a pH of 6.4 to 6.6.

8. The method according to claim 1, wherein in the step S4, a temperature for performing the primary recrystallization is -20 °C to 37 °C; in the step S5, a temperature for performing the secondary recrystallization is -20 °C to 25 °C.

9. The method according to claim 1, wherein the method includes at least one of the following (1) to (5):
(1) in the step S1, a mass ratio of ginsenoside Rb1 to the phosphate buffer solution is 1:(2 to 20), and a concentration of the phosphate buffer solution is 0.5 to 1.5 mM;
(2) in the step S1, a mass ratio of the complex enzyme to the phosphate buffer solution is 1:(10 to 20), and the concentration of the phosphate buffer solution is 0.5 to 1.5 mM;
(3) in the step S2, a mass concentration of the ethanol solution is 40% to 95%, and a mass ratio of the precipitate to the ethanol solution is 1:(8 to 12);
(4) in the step S2, prior to a plate-and-frame pressure filtration, the dissolved solution is mixed with a diatomaceous earth under stirring, followed by the pressure filtration, wherein a mass ratio of the dissolved solution to the diatomaceous earth is 1:(0.01 to 0.1); and
(5) in the step S2, a filtration pore size of the microfiltration is 0.22 µm.

10. Use of the preparation method according to any one of claims 1 to 9 in preparation of the rare ginsenoside CK.
